# EUROPEAN PATENT SPECIFICATION

(11) **EP 1 763 338 B1**
(45) Date of publication and mention of the grant of the patent: **05.01.2011**
(21) Application number: 05785782.3
(22) Date of filing: 27.06.2005
(51) Int. Cl.: A61K 9/20, A61P 9/06

(54) **ORAL SUSTAINED RELEASE FORMULATION OF TEDISAMIL WITH GASTRIC RETENTION PROPERTIES**
ORALE TEDISAMILFORMULIERUNGEN MIT MAGENRETENTIONSEIGENSCHAFTEN UND VERZÖGERTER FREISETZUNG
FORMULATION ORALE DE TEDISAMIL À LIBÉRATION PROLONGÉE AVEC DES PROPRIÉTÉS DE RÉTENTION GASTRIQUE

(30) Priority: 28.06.2004 US 582798 P; 28.06.2004 EP 04102987
(43) Date of publication of application: 21.03.2007
(73) Proprietor: Abbott Healthcare Products B.V., 1381 CP Weesp (NL)
(72) Inventor: BAKKER, Johan A. Solvay Pharmaceuticals B.V., 1381 CP Weesp (NL); DE WINTER, Marius L. Solvay Pharmaceuticals B.V., 1381 CP Weesp (NL); FABIANO, Santa Solvay Pharmaceuticals B.V., 1381 CP Weesp (NL)
(74) Representative: Verhage, Marinus
(86) International application number: PCT/EP2005/052984
(87) International publication number: WO 2006/000583

(56) References cited:
- WO-A-02/26214
- SINGH B N ET AL: "Floating drug delivery systems: an approach to oral controlled drug delivery via gastric retention" JOURNAL OF CONTROLLED RELEASE, ELSEVIER, AMSTERDAM, NL LNKD- DOI:10.1016/S0168-3659(99)00204-7, vol. 63, no. 3, 3 February 2000 (2000-02-03), pages 235-259, XP004244475 ISSN: 0168-3659
- DATABASE MEDLINE [Online] US NATIONAL LIBRARY OF MEDICINE (NLM), BETHESDA, MD, US; August 1993 (1993-08), TIMMERMANS J ET AL: "The cutoff size for gastric emptying of dosage forms." Database accession no. NLM8377128 & JOURNAL OF PHARMACEUTICAL SCIENCES AUG 1993, vol. 82, no. 8, August 1993 (1993-08), page 854, ISSN: 0022-3549
- SINGH B.N.; KIM K.H.: 'Floating drug delivery systems: an approach to oral controlled drug deilvery via gastric retention' J. CONTROLLED RELEASE vol. 63, 2000, pages 235 - 259

## Description

### FIELD OF THE INVENTION

The present invention relates to a novel sustained release formulation with gastric retention properties comprising tedisamil or a pharmaceutically acceptable salt thereof and the use of this formulation in the prevention and treatment of atrial fibrillation, atrial flutter and cardiac ischemia.

### BACKGROUND OF THE INVENTION

Tedisamil, a compound in development as its sesquifumarate salt, is a potassium channel blocking compound with a limited therapeutic window chemically described as 3',7'-bis(cydopropylmethyl)-spiro[cyclopentane-1,9'[3,7]diazabicydo-[3.3.1]-nonane], (2E)-2-butenedioate(2:3)

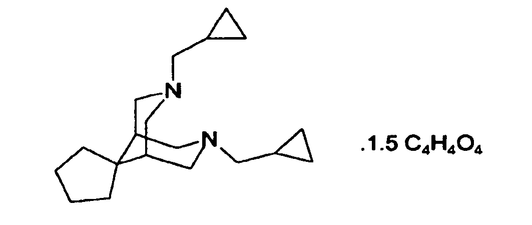

The compound has been shown to be effective in the conversion of atrial fibrillation to normal sinus rhythm and maintenance of sinus rhythm following cardio-version. It also possesses anti-ischemic properties. Tedisamil acts as a blocker of myocardial potassium outward channels and at higher concentrations also of sodium channels. It does not show negative inotropic effect and it does not influence the serum potassium levels. Moreover in clinical studies, it has been safely administered with commonly used drugs and warfarin. Also digitalls, atenolol and glibenc lamide had no clinically relevant effect on the pharmacokinetics of tedisamil.

### SUMMARY OF THE INVENTION

During clinical investigations it appeared that tedisamil administered in the form of immediate release tablets had some adverse effects, most notably gastrointestinal adverse events like diarrhea and abdominal pain which are probably caused by high peak plasma concentrations of the compound when using an immediate release formulation. Further it was concluded that low trough concentrations are expected to be responsible for limited efficacy, especially in the maintenance of sinus rhythm following cardio-version.

As a first solution to the above mentioned problems modified release formulations as described in WO 02/26214 were developed in order to lower the peak plasma concentrations. WO 02/26214 discloses sustained release formulations e.g. for tedisamil, having a substantially ion-strength independent release upon exposure to gastrointestinal fluids. It appeared, however, that the total availability of the active compound in these sustained release formulations was not sufficient (57% compared to immediate release formulation).

It is the object of the present invention to provide a formulation yielding less gastrointestinal side effects and a bioavallability of at least 70% compared to the immediate release formulation and preferably at least 75%.

It has surprisingly been found that the above mentioned objects can be obtained with a sustained-release formulation of tedisamil having gastric retention properties. This is especially surprising in view of the fact that the absorption of tedisamil from the proximal gastrointestinal tract is much worse than the absorption from the distal Intestinal tract (ileum).

### DETAILED DESCRIPTION OF THE INVENTION

The present invention is related to a sustained-release formulation having gastric retention properties. These gastric retention properties can be obtained by using a floating drug delivery system as described by B.N. Singh and K.H. Kim (J. Controlled Release 2000, 63, 235-259), preferably having a density of less than 1.0 g/cm³, or using a swelling and expanding delivery system, i.e. a formulation that swells in the stomach to such an extent that it can no longer pass the pyloric sphincter. (ref: J. Timmermans and A.J. Moes, The cut-off size for gastric emptying of dosage forms, J. Pharm. Sci. 1993, 82 (8), 854).

Sustained-release is defined as a (gradual) release of the active substance from the dosage form over a time period of 2 to 8 hours or more. This period starts usually with the administration of the dosage form, or with the start of in -vitro dissolution test (the moment the dosage form is brought into the dissolution medium). In the framework of the present invention sustained -release formulations are compared with immediate release formulations wherein the active substance is mainly (i.e. at least 80%) released within 45 minutes from the administration of the dosage form or form the start of the in-vitro dissolution test.

In an embodiment of the invention the drug delivery system is both floating and swelling. It comprises 5 to 40 wt % (preferably 10 to 30 wt %) of tedisamil or a pharmaceutically acceptable salt thereof, 30 to 85 wt % of a hydroswelling polymer and 2.5 to 5 wt % of a salt being capable of releasing gaseous carbon dioxide in a gastric environment, said formulation having both floating and swelling properties leading to gastric retention. A swelling enhancer is added at 0.5 to 10 wt%, (preferably 1-4 wt% and most preferred 3%). The swelling enhancer is alginic acid.

Although a coating is not essential to achieve the desired effect of the present invention, the formulation is optionally coated with a coating material in order to achieve another desired effect, such as masking of the taste or application of color. Suitable coating materials are known in the art and are for example HPMC , acrylics, ethylcellulose (see Graham Cole ed., Pharmaceutical Coating Technology, London, Taylor & Francis Ltd.; 1995).

There are several hydroswelling polymers that can be used to obtain a sustained-release formulation. Examples of hydroswelling polymers are hydroxypropyl methylcellulose (HPMC) hydroxylpropyl cellulose (HPC), hydroxyethylcellulose (HEC), carboxymethylcellulose (CMC).

The cellulose ethers that can be used in the present invention are well known in the art and are available in pharmaceutical grades and with different average molecular weights leading to different viscosities of a solution of these cellulose ethers. For the purpose of this patent application, hydrophilic polymers may be characterized by their viscosities in a 2% w/w aqueous solution as low viscosity (less than about 1000 mPas), medium viscosity (about 1000 mPas to about 10,000 mPas) and high viscosity (greater than about 10,000 mPas)

Hydrophilic hydroxypropyl methylcellulose polymers (HPMC's) which may be used In the present invention are available in different viscosity grades from Dow Chemical Co. under the brand name Methocel® and from Shin Etsu under the brand name MetoloseⓇ.

Examples of low viscosity polymers are Methocel E5®, Methocel E-15LV®, Methocel E50LV®, Methocel K100LV® and Methocel F50LV®, whose 2% aqueous solutions at 25°C have viscosities of 5 mPas, 15 mPas, 50 mPas, 100 mPas and 50 mPas, respectively.

Examples of medium viscosity HPMC's are Methocel E4M® and Methocel K4M, whose 2% aqueous solutions at 25°C have viscosities of 4000 mPas.

Examples of high viscosity HPMC's are Methocel K15M® and Methocel K100M® whose 2% aqueous solutions at 25 C have viscosities of 15,000 mPas and 100,000 mPas.

Hydrophilic hydroxyethylcellulose polymers (HEC's) which may be used in the present invention are available in different viscosity grades from AQUALON under the brand name Natrosol® and from Amerchol Corporation under the brand name Cellosize®.

Examples of low viscosity polymers are Natrosol L® en Natrosol J®, whose 2% aqueous solutions at 25 °C have viscosities of 10 mPas and 20 mPas, respectively. Examples of medium viscosity polymers are Natrosol G® and Natrosol K® whose 2% aqueous solutions at 25 °C have viscosities of 200 mPas and 1500 mPas, respectively.

Examples of high viscosity polymers are Natrosol M® and Natrosol HH® whose 2 % aqueous solutions have viscosities at 25 °C of 4000 mPas and 90000 mPas, respectively.

In a preferred embodiment of the present invention the formulation comprises a mixture of a high or medium viscosity hydroxypropylmethylcellulose (HPMC) and a high or medium viscosity hydroxyethylcellulose (HEC) as described in WO 02/26214. The ratio between the high or medium viscosity HPMC and the high or medium viscosity HEC is 1/0.85 to 1/1.2, preferably is 1/0.9 to 1/1.1, even more preferably is 1/0.95 to 1/1.05 and most preferred is 1/1. The formulation optionally may comprise a low viscosity HPMC. In that case the ratio between high or medium viscosity HPMC and low viscosity HPMC is in the range between 1/0.01 and 1/0.2 and preferably is between 1/0.01 and 1/0.1 and even more preferably is between 1/0.02 and 1/0.05.

As a salt being capable of releasing gaseous carbon dioxide alkaline metal carbonates can be used, preferably sodium carbonate potassium carbonate or sodium bicarbonate. An acid may be added, such as citric acid and maleic acid. The preferred carbonate is sodium bicarbonate and the preferred acid is citric acid.

Further auxiliary materials may be acid such as lubricants (e.g. sodium stearyl fumarate (Pruv®), glycerol behenate (Compritol 888 ATO), glidants (e.g. colloidal silicon dioxide (Aerosil®)), disintegrant (e.g. sodium starch glycolate (Primojel®)). fillers (e.g. mannitol), colorants and anti-oxidants.

The present invention also relates to a method of preparing a formulation as described above, characterized in that
(1) a core is compressed of a mixture comprising 5 to 40 wt % of tedisamil or a pharmaceutically acceptable salt thereof, 30 to 85 wt % of a hydroswelling polymer matrix and 2.5 to 5 wt % of a salt being capable of releasing gaseous carbon dioxide in a gastric environment and optionally 0.5 to 10 wt % of a swelling enhancer.
(2) the core is optionally coated.

In the preferred embodiment the ingredients HPMC (K4M and E5) and HEC are mixed. The mixture obtained is blended with a mixture of premilled citric acid and sodium bicarbonate and alginic acid. Milled tedisamil sesquifumarate is added and mixed. The lubricant is sieved and added followed by mixing with the fill er. The active substance may be added in the form of a pregranulate to the powder mixture used to compress. Alternatively the powder mixture for tabletting may be produced by a mixing procedure that is followed by a (wet or dry) granulation process.

The mixture of ingredients is compressed into tablets with commercial available equipment (e.g. a Courtoy® RO) using flow regulating agents like colloidal silica and lubricating agents like talcum, sodium stearyl fumarate or magnesium stearate. The quantity of hydrophilic celluloses in the complete formulation ranges between 30% and 85 %, preferably between 33% and 70%, while the amount of tedisamil or tedisamil salt ranges between 5% and 40%, preferably between 10 and 30%. The amount of flow regulating and lubricating agent is fixed to improve powder flow properties and to prevent sticking of powder to the dye walls or the punches. The amount of glidant is between 0 wt % and 5 wt %, preferably between 2 and 5% and is most preferably about 3 wt %. The amount of lubricant is between 2 wt % and 4 wt % and is preferably about 3 wt %. To achieve taste masking, the tablet core may be coated with 2 - 4 % w/w of a polymer mixture with or without pigments. Typical coating blends are commercially available, e.g. from COLORCON® as Opadry.®.

The present invention can be used to administer the active compound tedisamil to a mammal, especially a human, in need thereof. Especially usefull indications for tedisamil are the prevention and treatment of atrial fibrillation, atrial flutter and cardiac ischemia.

The present invention is therefore also related to the use of tedisamil or a pharmaceutically acceptable salt thereof, in the manufacure of a gastric retentive dosage form for the prevention or treatment of atrial fibrillation, atrial flutter or cardiac ischemia in a mammal, and more especially in a human.

### Reference Example 1. Preparation of non-coated Modified Release formulations formulations with an in vitro 100% dissolution in 4-6 hours containing 150 mg tedisamil in the form of its sesquifumarate.

A batch size of 1.5 kg, equivalent to 4286 tablets, was made with the following composition:

| Component | Quantity (g) |
|---|---|
| tedisamil sesquifumarate | 1028 |
| HPMC (K4M) | 193 |
| HPMC (E5) | 39 |
| HEC (HX250PH) | 193 |
| colloidal anhydrous silica | 26 |
| sodium stearyl fumarate | 21 |

- The required quantities of tedisamil sesquifumarate. HPMC K4M, HPMC E5, HEC HX250 PH and sieved colloidal anhydrous silica are mixed.
- The required quantity of sodium stearyl fumarate is sieved and mixed with the tedisamil mixture.
- The final powder mixture is compressed into tablets (target core weight: 350 mg).

### Reference Example 2. Preparation of coated Modified Release formulations formulations with an in vitro 100% dissolution In 8-12 hours containing 150 mg tedisamil in the form of its sesquifumarate.

A batch size of 1.5 kg, equivalent to 3529 tablets, was made with the following composition:

| Component | Quantity (g) |
|---|---|
| *tablet core* | |
| tedisamil sesquifumarate | 849 |
| HPMC (K4M) | 285 |
| HPMC (E5) | 49 |
| HEC (HX250PH) | 285 |
| colloidal anhydrous silica | 14 |
| sodium stearyl fumarate | 18 |
| *coating* | |
| polyacrylate dispersion 30% m/m ¹) | 73.5 |
| HPMC (E5) | 2.2 |
| talc (micro talc) | 11 |
| purified water | about 417 |

| | |
|---|---|
| ¹) e.g. Eudragit NE30D® of Röhm Pharma | |

The weight increase due to application of coating is approx. 10 mg/tablet.

The manufacture procedure is as follows:
- The required amount of colloidal anhydrous silica is sieved.
- The required quantities of tedisamil sesquifumarate, HPMC K4M, HPMC E5, HEC HX250 PH and sieved colloidal anhydrous silica are mixed.
- The required quantity of sodium stearyl fumarate is sieved and mixed with the tedisamil mixture.
- The final powder mixture is compressed into tablets (target core weight: 425 mg).
- The tablets are coated by spraying a coating suspension consisting of polyacrylate dispersion 30% m/m, HPMC E5 and talc suspension in water on the tablets (target tablet weight: 435 mg).

### Example 3. Preparation of floating expanding formulations containing 138 mg tedisamil in the form of its sesquifumarate.

**A batch size of 1.0 kg, equivalent to 1351 tablets, was made with the following** composition:

| Component | Quantity (g) |
|---|---|
| tedisamil sesquifumarate | 299.1 |
| mannitol (25) | 200 |
| HPMC (K4M) | 193 |
| HEC (HX250PH) | 193 |
| glyceryl behenate (Compritol 888) | 40.5 |
| alginic acid | 37.8 |
| sodium bicarbonate | 24.3 |
| citric acid anhydrous | 6.76 |
| HPMC (E5) | 6.08 |

The manufacture procedure is as follows:
- The required quantities of HPMC (K4M), HPMC (E5) and HEC are mixed.
- The citric acid anhydrous is milled, and added to the mixture mentioned above. The sodium bicarbonate anhydrous and alginic acid are added, and the powders are mixed.
- The milled tedisamil sesquifumarate is added and mixed.
- The glyceryl behenate is sieved through a 0.5-mm screen, and added. The mannitol is added, and the powders are mixed.
- The final powder mixture is compressed into tablets (target tablet weight: 740 mg).

### Reference Example 4. Preparation of Immediate release formulations containing 119.7 mg tedisamil In the form of its dihydrochloride.

A batch size of 144.8 kg, equivalent to 800,000 tablets, was made with the following composition:

| Component | Quantity (kg) |
|---|---|
| tedisamil dihydrochloride | 60.00 |
| lactose monohydrate | 60.00 |
| stearic palmitic acid | 6.00 |
| talc | 5.40 |
| colloidal anhydrous silica | 0.60 |
| Opadry Y-1 7000 White | 14.08 |

The manufacture procedure is as follows:
- The required quantities of tedisamil dihydrochloride, lactose monohydrate, talc, and colloidal anhydrous silica are mixed
- The stearic palmitic acid is added, and the powders are mixed
- The final powder mixture is compressed into tablets (target tablet weight: 165 mg).
- The tablets are coated by spraying a coating suspension of Opadry Y-I7000 White in water on the tablets (target tablet weight: 181 mg).

### Example 5. Single dose partially balanced cross-over study comparing two different modified release (MR) formulations of a tedisamil salt, an immediate release (IR) formulation and a floating expending (FE) formulation.

Tedisamil was given to 20 healthy male volunteers in the age range of 18 to 45 years. Each subject was scheduled to receive three of the following single dose formulations:
A: formulation as described in Example 1 (15 subjects have analyzable data).
B: formulation as described in Example 2 (14 subjects have analyzable data).
D: formulation as described in Example 3 (13 subjects have analyzable data).
E: two times the formulation as described in Example 4 (15 subjects have analyzable data).

The plasma concentrations were measured and adverse events information was assessed using non-leading questions

### Pharmacokinetic data

As presented in Table 1, the dose-normalized pharmacokinetic parameters showed that the FE tablet had the highest exposure compared to the IR reference (shaded), and was therefore selected for the multiple dose study.

**Table 1: Pharmacokinetic data**

| **Pharmacokinetics tedisamil free base** | | | | | | |
|---|---|---|---|---|---|---|
| **Treatment** | **n** | **Tₘₐₓ h** | **Cₘₐₓ ng/ml** | **AUC₀₋ₜ ng*h/ml** | **AUC ng*h/ml** | **T½ h** |
| E119.7mg | 15 | 1.47 | 723 | 3700 | 3770 | 10.8 |
| A 150 mg | 15 | 3.23 | 361 | 3000 | 3050 | 8.26 |
| Ratio A/E | | | 0.398 | 0.647 | 0.646 | |
| B 150 mg | 14 | 3.64 | 388 | 3080 | 3190 | 10.5 |
| Ratio B/E | | | 0.428 | 0.664 | 0.675 | |
| D 138mg | 13 | 3.54 | 347 | 3250 | 3330 | 9.23 |
| Ratio D/E | | | 0.416 | 0.762 | 0.766 | |

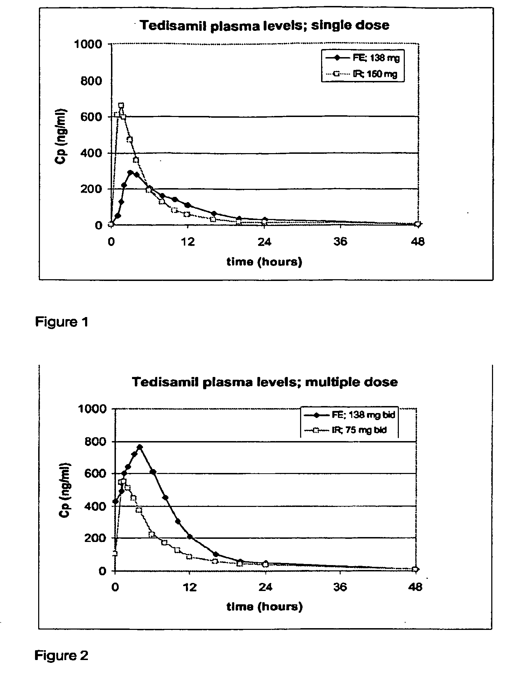

The actual plasma levels achieved (group means) by the single doses of FE 138 mg (formulation D) and IR 119.7 mg (formulation E) tedisamil free base are illustrated in Figure 1. The resulting exposure was slightly higher (AUC 113%; AUC is Area under the Curve (0→ infinity)) for the IR compared to the FE formulation, and a twice as high peak plasma level for the IR compared to the FE formulation.

### Tolerability

Tedisamil is known to cause gastro-intestinal adverse events, most notably diarrhea-type complaints, and this was confirmed in this study for the IR tablet. After a single dose of 119.7 mg IR, six out of 15 subjects complained of 10 gastro-intestinal adverse events (diarrhea 6x, abdominal pain 4x). Of the 10 gastro-intestinal events, 6 were scored to be moderately severe. After a single dose of 138 mg FE, three out of 13 subjects complained of gastro-intestinal adverse events (tongue disorder 2x, abdominal pain 1x. The tongue disorder was scored to be moderately severe once. The treatment emergent adverse events are presented in Table 2.

**Table 2. Gastro intestinal adverse effects in single dose study comparing IR formulation with FE formulation.**

| **MedDRA SOC** | **Preferred term** | **FE 138 mg (n=13)** | | | **IR 119.7 mg (n=15)** | | |
|---|---|---|---|---|---|---|---|
| | | **N** | **%** | **n** | **N** | **%** | **n** |
| **Gastrointestinal** | **Any** | **2** | **15.4** | **3** | **6** | **40** | **10** |
| **disorders** | Abd pain NOS¹ | 1 | 7.7 | 1 | 1 | 6.7 | 2 |
| | Abd pain upper | | | | 2 | 13.3 | 2 |
| | Diarrhea NOS¹ | | | | 5 | 33.3 | 6 |
| | Tongue disorder NOS¹ | 1 | 7.7 | 2 | | | |

| | | | | | | | |
|---|---|---|---|---|---|---|---|
| N: number of subject with event %: proportion of exposed subjects having the event n: number of events 1) NOS = Not otherwise specified | | | | | | | |

### Example 6. Multiple dose study comparing an Immediate release (IR) formulation and a floating expending (FE) formulation of a tedisamil salt.

Tedisamil was given to healthy male volunteers in the age range of 18 to 4 5 years. A total of 18 subjects was divided into 2 parallel groups receiving the following single dose formulations during 7 days:
D1: two times per day the formulation: as described in Example 3 (9 subjects).
E1: two times per day the formulation as described in Example 4 (9 subjects).

### Pharmacokinetic data

In the multiple dose study part, a plasma curve was taken at steady state following a morning dose. The dose-normalized exposure (AUC) after the FE formulation was about 80% of the exposure to the IR tablet, and the Cₘₐₓ was about 60%, see Table 3. (From both groups a complete PK curve was available from 8 subjects).

**Table 3. Pharmacokinetic data at steady state.**

| **Pharmacokinetics tudisamil free base** | | | | | |
|---|---|---|---|---|---|
| **Treatment** | **n** | **Tₘₐₓ h** | **Cₘₐₓ ng/mt** | **AUCₛₛ ng*h/ml** | **T½ h** |
| E1: 59.58 mg BID | 8 | 1.5 | 589 | 3260 | 12.3 |
| D1: 138 mg BID | 8 | 3 | 798 | 6250 | 11 |
| Ratio FE/IR | | | 0.588 | 0.832 | |

| | | | | | |
|---|---|---|---|---|---|
| BID = twice per day ss = steady state | | | | | |

The actual doses given were very different: the FE tablet was dosed at two times 138 mg free base dally and the IR tablets at two times 59.58 mg free base daily. This resulted in about 90% higher exposure and 35% higher peak plasma levels (group means) for the FE than for the IR formulation, as illustrated in the Figure 2.

### Tolerability MD

The single dose tolerability results were confirmed in this study for the IR tablet, where six of the nine subjects complained of 23 gastro-intestinal side effects (flatulence 9x, loose stools 4x, diarrhea 4x, watery stools 1x, abdominal pain 4x, defecation urgency 1x). The flatulence and loose stools were each reported to be moderately severe once and the abdominal pain twice. In contrast, on the FE tablet only three of the nine subjects complained of three gastro-intestinal side effects (flatulence 1x, loose stools 1x, diarrhea 1x). All were mild. Few adverse events were classified in other system organ classes, and this was more or less evenly distributed in the two groups.

All treatment emergent gastro-intestinal adverse events are presented in Table 4.

**Table 4. Gastro intestinal adverse effects in multiple dose study comparing IR formulation with FE formulation.**

| **MedDRA SOC** | **Preferred term** | **FE 276 mg (n=9)** | | | **IR 1 t9.7 mg (n=9)** | | |
|---|---|---|---|---|---|---|---|
| | | **N** | **%** | **n** | **N** | **%** | **n** |
| **Gastrointestinal disorders** | **Any** | 3 | 33.3 | 3 | 6 | 66.7 | 23 |
| | Abd pain NOS | | | | 2 | 22.2 | 2 |
| | Abd pain upper | | | | 1 | 11.1 | 2 |
| | Defecation urgency | | | | 1 | 11.1 | 1 |
| | Diarrhea NOS | 1 | 11.1 | 1 | 2 | 22.2 | 4 |
| | Flatulence | 1 | 11.1 | 1 | 5 | 55.6 | 9 |
| | Loose stools | 1 | 11.1 | 1 | 3 | 33.3 | 4 |
| | Stools watery | | | | 1 | 11.1 | 1 |

| | | | | | | | |
|---|---|---|---|---|---|---|---|
| N: number of subject with event %: proportion of exposed subjects having the event n: number of events | | | | | | | |

### Conclusion

Tedisamil as a salt in the floating expending (FE) tablet is much better tolerated than In the immediate release formulation with regard to the gastro-intestinal adverse effects. In the data presented the difference was especially striking under multiple dose conditions, despite the fact that the exposure was almost twice as high in the FE group.

## Claims

1. A sustained-release formulation of tedisamil, having gastric retention properties caused both by floating on the gastric fluid, and swelling and expanding of the formulation in the gastric fluid, comprising 5 to 40 wt % tedisamil, or a pharmaceutically acceptable salt thereof, 30 to 85 wt % of a hydroswelling polymer matrix, 2.5 to 5 wt % of a salt being capable of releasing gaseous carbon dioxide in a gastric environment, and 0.5 to 10 wt% alginic acid.

2. A formulation as claimed in claim 1, wherein said pharmaceutically acceptable salt is the sesquifumarate salt, or the dihydrochloric acid salt.

3. A formulation as claimed in claim 1 or claim 2, wherein the salt being capable of releasing gaseous carbon dioxide in a gastric environment, is selected from sodium carbonate, potassium carbonate and sodium bicarbonate.

4. A method of preparing a formulation according to any of the claims 1-3, wherein:
(1) a core is compressed of a mixture comprising 5 to 40 wt % of tedisamil, or a pharmaceutically acceptable salt thereof, 30 to 85 wt % of a hydroswelling polymer matrix, 2.5 to 5 wt % of a salt being capable of releasing gaseous carbon dioxide in a gastric environment, and 0.5 to 10 wt % alginic acid,
(2) the core is optionally coated.

5. Use of a formulation according to any of 1-3, for the preparation of a medicament for the prevention and treatment of atrial fibrillation, atrial flutter, or cardiac ischemia.

## Patentansprüche

1. Formulierung von Tedisamil mit verzögerter Freisetzung, welche Magenretentionseigenschaften aufweist, welche sowohl durch das Schwimmen auf dem Magensaft als auch durch das Aufquellen und sich Ausdehnen der Formulierung im Magensaft entstehen, umfassend 5 bis 40 Gew.% Tedisamil oder eines pharmazeutisch akzeptablen Salzes davon, 30 bis 85 Gew.% einer in Wasser aufquellenden Polymermatrix, 2,5 bis 5 Gew.% eines Salzes, welches in der Lage ist, in einer Magenumgebung gasförmiges Kohlendioxid freizusetzen, und 0,5 bis 10 Gew.% Alginsäure.

2. Formulierung nach Anspruch 1, wobei das pharmazeutisch akzeptable Salz das Sesquifumarat-Salz oder das Dichlorwasserstoffsäure-Salz ist.

3. Formulierung nach Anspruch 1 oder 2, wobei das Salz, welches in der Lage ist, in einer Magenumgebung gasförmiges Kohlendioxid freizusetzen, aus Natriumcarbonat, Kaliumcarbonat und Natriumbicarbonat ausgewählt ist.

4. Verfahren zur Herstellung einer Formulierung nach einem der Ansprüche 1-3, wobei:
(1) ein Kern aus einer Mischung umfassend 5 bis 40 Gew.% Tedisamil oder eines pharmazeutisch akzeptablen Salzes davon, 30 bis 85 Gew.% einer in Wasser aufquellenden Polymermatrix, 2,5 bis 5 Gew.% eines Salzes, welches in der Lage ist, in einer Magenumgebung gasförmiges Kohlendioxid freizusetzen, und 0,5 bis 10 Gew.% Alginsäure komprimiert wird,
(2) der Kern wahlweise überzogen ist.

5. Verwendung einer Formulierung nach einem der Ansprüche 1-3 zur Herstellung eines Medikamentes zur Prävention und Behandlung von Vorhofflimmern, Vorhofflattern oder kardialer Ischämie.

## Revendications

1. Formulation à libération prolongée de tedisamil ayant des propriétés de rétention gastrique provoquées à la fois par flottaison sur le liquide gastrique et gonflement et expansion de la formulation dans le liquide gastrique, comprenant 5 à 40 % en poids de tedisamil ou d'un sel pharmaceutiquement acceptable de ce dernier, 30 à 85 % en poids d'une matrice polymère hydrogonflante, 2,5 à 5 % en poids d'un sel capable de libérer du dioxyde de carbone gazeux dans un environnement gastrique et 0,5 à 10 % en poids d'acide alginique.

2. Formulation selon la revendication 1, où ledit sel pharmaceutiquement acceptable est le sel sesquifumarate ou le sel de l'acide dichlorhydrique.

3. Formulation selon la revendication 1 ou la revendication 2, où le sel capable de libérer du dioxyde de carbone gazeux dans un environnement gastrique est sélectionné parmi le carbonate de sodium, le carbonate de potassium et le bicarbonate de sodium.

4. Procédé de préparation d'une formulation selon l'une quelconque des revendications 1 à 3, où :
(1) on compresse un noyau d'un mélange comprenant 5 à 40 % en poids de tedisamil ou d'un sel pharmaceutiquement acceptable de ce dernier, 30 à 85 % en poids d'une matrice polymère hydrogonflante, 2,5 à 5 % en poids d'un sel capable de libérer du dioxyde de carbone gazeux dans un environnement gastrique et 0,5 à 10 % en poids d'acide alginique,
(2) le noyau est facultativement enrobé.

5. Utilisation d'une formulation selon l'une quelconque des revendications 1 à 3 pour la préparation d'un médicament destiné à la prévention et au traitement de la fibrillation auriculaire, du flutter auriculaire ou de l'ischémie cardiaque.
